# EUROPEAN PATENT APPLICATION

(11) **EP 3 705 110 A1**
(43) Date of publication of application: **09.09.2020**
(21) Application number: 18872021.3
(22) Date of filing: 01.11.2018
(51) Int. Cl.: A61K 8/49, A23L 33/10, A61K 31/352, A61Q 19/02

(54) **COMPOSITION FOR IMPROVING SKIN WHITENING OR AMELIORATING THERMAL SKIN AGING COMPRISING 8-METHOXYBUTIN**

(30) Priority: 01.11.2017 KR 20170144689
(71) Applicant: Hesed Bio Co., Ltd., Pohang-si Gyeongsangbuk-do 37673 (KR)
(72) Inventor: KIM, Kyong Tai, Pohang-si Gyeongsangbuk-do 37673 (KR); KIM, Hyo Jin, Daegu 41097 (KR); OH, Eunji, Seoul 02543 (KR); BAEK, Nam In, Hwaseong-si Gyeonggi-do 18441 (KR); KIM, Hyoung-Geun, Daegu 42750 (KR)
(74) Representative: Dehns
(86) International application number: PCT/KR2018/013172
(87) International publication number: WO 2019/088727

(57) **Abstract**

The present invention relates to a composition for improving skin whitening or thermal aging comprising 8-methoxybutin or an acceptable salt thereof as an active ingredient. The composition has no cytotoxicity, increases the expression of heat shock protein 70 (HSP70)-to prevent thermal aging, and inhibits the melanogenesis by decreasing level of tyrosinase gene expression involved in skin pigmentation. Therefore, the composition including 8-methoxybutin or an acceptable salt thereof as an active ingredient can be effectively used as a cosmetic composition, dermatologic agent, health functional food, and the like for improving skin whitening or thermal aging.

## Description

### TECHNICAL FIELD

The present invention relates to a composition for improving skin whitening or thermal aging comprising 8-methoxybutin or an acceptable salt thereof as an active ingredient, and more particularly to a cosmetic composition for improving skin whitening or thermal aging, comprising 8-methoxybutin or an acceptable salt thereof as an active ingredient, a dermatologic agent for improving skin whitening or thermal aging, a health functional food for improving skin whitening or thermal aging, a pharmaceutical composition for improving skin whitening or thermal aging, a quasi-drug composition for improving skin whitening or thermal aging, and a method of improving skin whitening or thermal aging, which comprises applying the composition onto the skin.

### BACKGROUND ART

Natural products have long been used as materials that have potential to be used in drugs, foods, and cosmetics. Natural materials derived from plants such as alkaloids, terpenes and flavonoids are known. Flavonoids consist of a group of polyphenol substances, and they are secondary metabolites of fruits, vegetables, medicinal plants. They play an important role in determining the color of flowers, fruits, and vegetables and protecting from UV absorption. Flavonoids are distinguished by oxidation or substitution of certain residues based on a 15-carbon structure in which two benzene rings are connected.

Flavonoids have been reported to have various biological activities such as anti-oxidant, liver protection, anti-bacterial, anti-viral and anti-cancer effects. Also, flavonoids have the potential to be used as a cosmeceutical material due to their excellent anti-aging activity and skin whitening activity.

The major cause of skin aging is the generation of heat and reactive oxygen species (ROS) by ultraviolet rays and infrared rays of the sun. In particular, infrared rays increase the temperature of the skin, which is maintained at an average temperature of 37 °C, to 40 °C or more, and cause heat shock, and increase pigmentation and wrinkle formation when a high temperature is continued. This is called thermal skin aging. According to recent studies, it has been reported that heat aging by infrared rays in addition to ultraviolet rays accelerates photo-aging (J. Dermatological Sci. Supplement (2006) 2, S13-S22 "Thermal aging: A new concept of skin aging", Photodermatol Photoimmunol Photomed (2006) 22, 148-152 "Minimal heating dose: a novel biological unit to measure infrared irradiation), and academia has great interest in this.

When stress is induced by continuous heat exposure in cells, the expression of the heat shock protein HSP70 increases and block skin damage caused by stress induction to protect the skin. Therefore, effective induction of HSP70 may improve skin aging due to heat.

The color of the skin is determined by the amount and distribution of melanin synthesized by melanocytes in the skin. The synthesis of the melanin pigment is regulated by an α-melanocyte stimulating hormone (α-MSH) signaling mechanism, and the binding of α-melanocyte stimulating hormone and the melanocortin-1 receptor (MC1R receptor) increases cyclic adenosine monophosphate (cAMP), thereby activating protein kinase A (PKA), leading to the activation of the microphthalmia-associated transcription factor (MITF), a transcription factor that produces tyrosinase, which is an important factor in the production of melanin. Tyrosinase converts tyrosine into DOPA and dopaquinone in melanocytes and produces melanin through non-enzymatic oxidation.

Recently, cosmetic industry has trended towards the development of multifunctional cosmetic materials having anti-thermal aging, skin whitening, and anti-oxidant activities. In addition, plant extracts that are highly safe and contain many active ingredients are used as cosmetic materials.

### DESCRIPTION OF EMBODIMENTS

### TECHNICAL PROBLEM

The inventors of the present invention had conducted research on discovering flavonoid ingredients exhibiting a skin whitening or thermal aging improvement effect, which are derived from natural products, and confirmed that 8-methoxybutin separated from *Coreopsis lanceolata* exhibited improved skin whitening or thermal aging activity, and thus completed the present invention.

### TECHNICAL SOLUTION

An object of the present invention is to provide a cosmetic composition for improving skin whitening or thermal aging, which comprises 8-methoxybutin or a cosmetically acceptable salt thereof as an active ingredient.

Another object of the present invention is to provide a dermatologic agent for improving skin whitening or thermal aging, which comprises 8-methoxybutin or an acceptable salt thereof as an active ingredient.

Still another object of the present invention is to provide a health functional food for improving skin whitening or thermal aging, which comprises 8-methoxybutin or a neutraceutically acceptable salt thereof as an active ingredient.

Yet another object of the present invention is to provide a pharmaceutical composition for improving skin whitening or thermal aging, which comprises 8-methoxybutin or a pharmaceutically acceptable salt thereof as an active ingredient.

Yet another object of the present invention is to provide a quasi-drug composition for improving skin whitening or thermal aging, which comprises 8-methoxybutin or a pharmaceutically acceptable salt thereof as an active ingredient.

Yet another object of the present invention is to provide a method of improving skin whitening or thermal aging, including applying, to the skin of an individual, a composition including 8-methoxybutin or a pharmaceutically acceptable salt thereof as an active ingredient.

### ADVANTAGEOUS EFFECTS OF INVENTION

According to the present invention, a composition comprising 8-methoxybutin as an active ingredient exhibits the effects of having no cytotoxicity, increasing the expression of heat shock protein 70 to prevent thermal agining and inhibiting the activity of tyrosinase involved in skin pigmentation. Thus the composition comprising 8-methoxybutin as an active ingredient can be effectively used as cosmetic composition, dermatologic agent, health functional food, and agents for improving skin whitening or thermal aging.

### BRIEF DESCRIPTION OF DRAWINGS

FIG. 1 is a graph showing the effect of 8-methoxybutin on toxicity and cell growth.
FIG. 2 is a set of graphs showing the inhibitory effect of 8-methoxybutin on tyrosinase and melanin synthesis.
FIG. 3 is a set of graphs showing the effect of 8-methoxybutin on increasing the expression of heat shock protein 70 when heat stress is induced.
FIG. 4 is a set of images showing the results of predicting the binding of 8-methoxybutin to an estrogen receptor through simulation.
FIG. 5 is a set of graphs showing that 8-methoxybutin has phytoestrogen activity through an estrogen receptor reporter experiment.

### BEST MODE

Each description and embodiment disclosed in the present invention may also be applied to other descriptions and embodiments. That is, all combinations of various elements disclosed in the present invention fall within the scope of the present invention. In addition, detailed descriptions set forth herein should not be construed as limiting the scope of the present invention.

In addition, those of ordinary skill in the art can recognize or identify numerous equivalents to certain aspects of the present invention described in the present application through only general experiments. In addition, these equivalents are intended to fall within the present invention.

Hereinafter, the present invention will be described in detail.

According to an embodiment of the present invention, there is provided a cosmetic composition for improving skin whitening or thermal aging, which comprises 8-methoxybutin or a cosmetically acceptable salt thereof as an active ingredient.

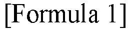

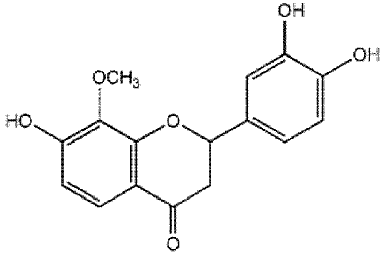

In the present invention, "8-methoxybutin" is a compound represented by Formula 1 above and having a molecular formula of C₁₆H₁₄O₆ and a molecular weight of 302 g/mol, which is also called flavanolanceoletin or 2-(3,4-dihydroxyphenyl)-7-hydroxy-8-methoxy-2,3-dihydro-4H-1-benzopyran-4-one. 8-methoxybutin may be extracted from *Coreopsis lanceolata* and it is not known to academia whether or not 8-methoxybutin can be extracted from chrysanthemums other than *Coreopsis lanceolata.* In addition, research on the specific pharmacological activity of 8-methoxybutin is still ongoing, but there is no report about whether 8-methoxybutin has an effect of improving skin whitening or thermal aging.

A method of obtaining 8-methoxybutin is not particularly limited, and 8-methoxybutin may be chemically synthesized using a method known in the art, or a commercially available material may be used.

The 8-methoxybutin represented by Formula 1, according to the present invention, may be present not only in a solvated form but also in an unsolvated form. The 8-methoxybutin of the present invention may be present in a crystalline or amorphous form, and all such physical forms fall within the scope of the present invention.

As used herein, the term "cosmetically acceptable salt" refers to a salt form in which 8-methoxybutin binds to other materials, and means a substance capable of exhibiting cosmetic activity similar to that of 8-methoxybutin.

In the present invention, "skin whitening" refers to not only brightening the skin tone by inhibiting the synthesis of melanin pigment, but also improving skin hyperpigmentation due to ultraviolet rays, hormones or heredity, such as spots or freckles.

The cosmetic composition of the present invention may be achieved by inhibiting the synthesis of melanin and inhibiting the activity of tyrosinase. Specifically, the cosmetic composition may inhibit the activity of tyrosinase, inhibit the generation of reactive oxygen species, or inhibit the generation of nitrate peroxide.

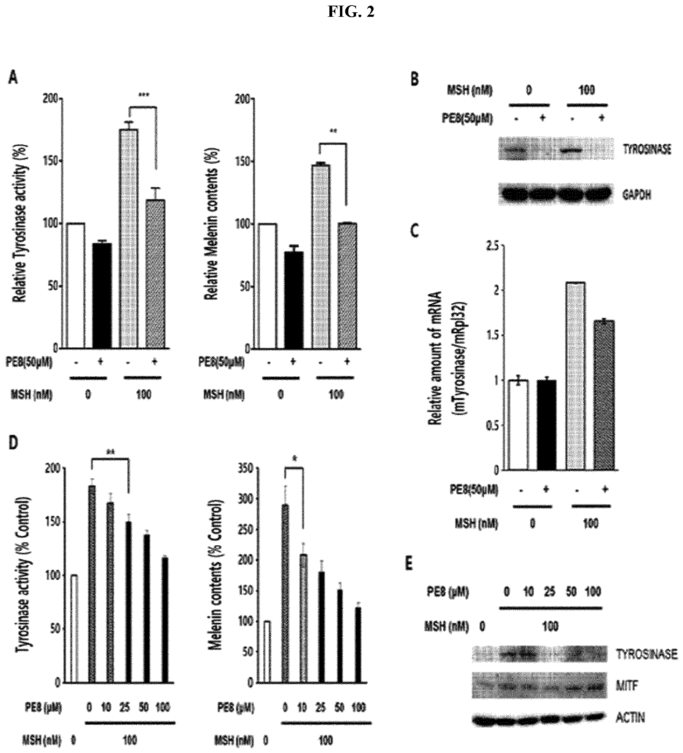

In one embodiment of the present invention, as a result of confirming the effect of 8-methoxybutin on inhibiting the activity of tyrosinase, which is associated with skin pigmentation, the compound exhibited an effect of reducing tyrosinase activity, particularly a significant effect of reducing tyrosinase activity as compared to GAPDH as a control, from which it was confirmed that the composition including 8-methoxybutin could be used as a cosmetic composition for skin whitening (see FIG. 2).

In addition, in another embodiment of the present invention, as a result of analyzing the effect of 8-methoxybutin on inhibiting the production of melanin, it was confirmed that the compound inhibited melanin synthesis in a concentration-dependent manner (see FIG. 2), from which it was reconfirmed that the composition including 8-methoxybutin could be used as a cosmetic composition for skin whitening.

In the present invention, the term "thermal aging" means thermal aging of the skin, refers to not only a physiological phenomenon in which the skin ages due to exposure to sunlight (ultraviolet rays), but also aging progression due to increased heat of the skin by infrared rays of 780 nm to 3,000 nm, which are relatively long wavelengths, and means that, when exposed to ultraviolet rays or infrared rays for a long time, deep wrinkles on the face and neck increase, and skin dryness and a rough skin surface, or pigmentation such as spots and freckles occur.

In the present invention, "improvement" includes inhibiting the degree of increase in wrinkles of the skin or alleviating roughness of the skin even when exposed to ultraviolet rays or infrared rays, and includes alleviating the degree of progression of pigmentation such as spots and freckles in the skin.

The cosmetic composition of the present invention may increase the expression of heat shock protein 70 (HSP70).

Heat shock proteins can be expressed under conditions of environmental stresses such as heat, amino acid analogues, heavy metals, toxins, and oxides, and under conditions of pathological stress such as when viruses or bacteria invade cells or when aging or inflammation is induced in cells. Among the heat shock proteins expressed against these various stresses, heat shock protein 70 is most responsive to temperature change, and when cells are subjected to a rapid temperature rise condition, heat shock protein 70 can be synthesized in cells within a few minutes. Such heat shock proteins are materials that protect cells under stress and increase the viability of cells under inappropriate conditions such as excessive external stimulation. Thereamong, the most important function of heat shock protein 70 as a molecular chaperone is to bind to other denatured enzyme proteins to maintain appropriate protein folding, thereby minimizing cell damage, and to play an important role in maintaining intracellular homeostasis such as protein transport or protein synthesis through cell membranes. Thus, as the expression of heat shock protein 70 increases, cell damage may be minimized to protect skin cells from thermal aging.

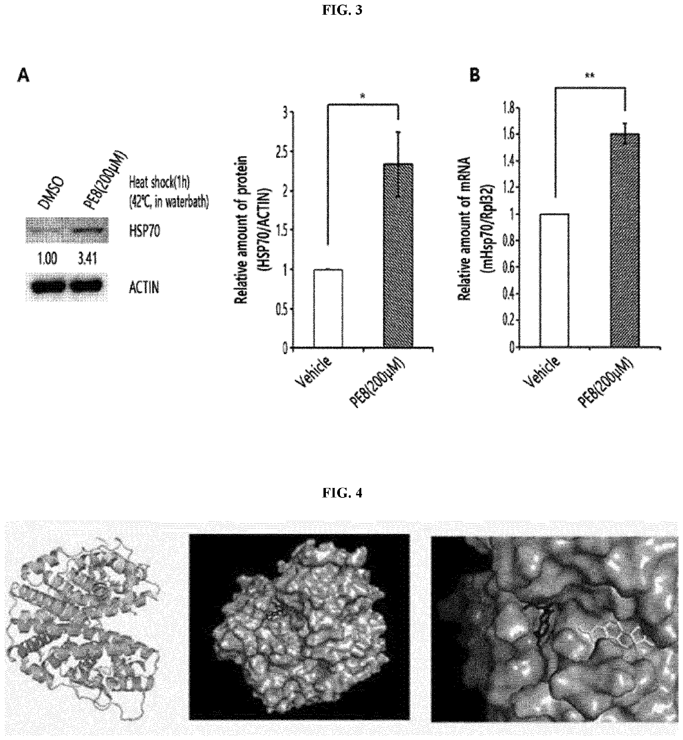

In one embodiment of the present invention, as a result of confirming the effect of 8-methoxybutin, which is associated with an improvement of thermal aging, on the expression of heat shock protein 70 (HSP70), the compound exhibited an effect of increasing the expression of heat shock protein 70, particularly a significant effect of increasing the expression of heat shock protein 70 as compared to ACTIN as a control, from which it was confirmed that the composition including 8-methoxybutin could be used as a cosmetic composition for improving thermal aging (see FIG. 3).

In the cosmetic composition of the present invention, 8-methoxybutin have phytoestrogenic activity. In the present invention, "phytoestrogenic activity" means that 8-methoxybutin binds to a phytoestrogen receptor so as to activate a phytoestrogen. When phytoestrogen is activated, skin thickness is maintained by preventing collagen loss, therefore, wrinkles do not occur and skin aging is delayed by maintaining skin moisture.

The cosmetic composition according to the present invention may be prepared into a formulation selected from the group consisting of a solution, an ointment for external use, a cream, a foam, a skin lotion, a skin softener, a pack, a skin toner, an emulsion, a makeup base, an essence, a soap, a liquid cleanser, a bath preparation, a sunscreen cream, suntan oil, a suspension, an emulsion, a paste, a gel, a lotion, a powder, a surfactant-containing cleanser, oil, a powder foundation, an emulsion foundation, a wax foundation, a patch, and a spray, but the present invention is not limited thereto.

In addition, the cosmetic composition according to the present invention may further include one or more cosmetically acceptable carriers that are mixed in general skin cosmetics, and general ingredients, for example, oil, water, a surfactant, a moisturizing agent, lower alcohols, a thickening agent, a chelating agent, a pigment, a preservative, and a fragrance may be appropriately mixed, but the present invention is not limited thereto.

The cosmetically acceptable carrier included in the cosmetic composition according to the present invention includes various formulations.

For ointment, paste, cream, or gel preparations of the present invention, as a carrier ingredient, animal oil, vegetable oil, wax, paraffin, starch, tragacanth, a cellulose derivative, polyethylene glycol, silicon, bentonite, silica, talc, zinc oxide, or a mixture thereof may be used.

For powder or spray preparations of the present invention, lactose, talc, silica, aluminum hydroxide, calcium silicate, polyamide powder, or a mixture thereof may be used as a carrier ingredient. In particular, in the case of spray preparations, the composition may further include a propellant such as a chlorofluorohydrocarbon, propane/butane, or dimethyl ether.

For solution or emulsion preparations of the present invention, a solvent, a solubilizing agent, or an emulsifying agent may be used as a carrier ingredient, and the carrier ingredient may be, for example, water, ethanol, isopropanol, ethyl carbonate, ethyl acetate, benzyl alcohol, benzyl benzoate, propylene glycol, or 1,3-butyl glycol oil. In particular, cottonseed oil, peanut oil, corn seed oil, olive oil, castor oil, sesame oil, glycerol fatty acid ester, or polyethylene glycol or sorbitan fatty acid ester may be used.

For suspension preparations of the present invention, as a carrier ingredient, a liquid diluent such as water, ethanol, or propylene glycol, a suspending agent such as ethoxylated isostearyl alcohol, polyoxyethylene sorbitol ester, or polyoxyethylene sorbitan ester, micro-crystalline cellulose, aluminum methahydroxide, bentonite, agar, tragacanth, or the like may be used.

For soap preparations of the present invention, as a carrier ingredient, an alkali metal salt of a fatty acid, a fatty acid hemiester salt, a fatty acid protein hydrolysate, isethionate, a lanolin derivative, a fatty alcohol, vegetable oil, glycerol, sugar, or the like may be used.

According to another embodiment of the present invention, there is provided a dermatologic agent for improving skin whitening or thermal aging, which comprises 8-methoxybutin or an acceptable salt thereof as an active ingredient.

As used herein, "8-methoxybutin" is the same as described above.

In the present invention, "acceptable salt" refers to a salt form in which 8-methoxybutin binds to other materials, and means a substance capable of exhibiting activity similar to that of 8-methoxybutin.

The term "dermatologic agent" as used herein is a concept of collectively encompassing all materials that are generally used for external application to the skin, and non-limiting examples of formulations of the dermatologic agent include plasters, lotions, liniments, liquids and solutions, aerosols, extracts, ointments, fluid extracts, emulsions, suspensions, capsules, creams, soft or hard gelatin capsules, patches, or sustained- release preparations.

In one embodiment of the present invention, as described above, 8-methoxybutin showed an effect of reducing tyrosinase activity, from which it was confirmed that the composition including 8-methoxybutin could be used as a dermatologic agent for skin whitening (see FIG. 2).

In another embodiment of the present invention, it was confirmed that 8-methoxybutin inhibited melanin synthesis in a concentration-dependent manner (see FIG. 2), from which it was reconfirmed that the composition including 8-methoxybutin could be used as a dermatologic agent for skin whitening.

In addition, in one embodiment of the present invention, as described above, 8-methoxybutin exhibited an effect of increasing the expression of heat shock protein 70, from which it was confirmed that the composition including 8-methoxybutin could be used as a dermatologic agent for improving skin thermal aging (see FIG. 3).

The dermatologic agent according to the present invention may be a preparation for parenteral administration, which is formulated in solid, semi-solid or liquid form by adding a compatible inorganic or organic carrier, excipient and diluent. The preparation for parenteral administration may be a transdermal dosage form selected from the group consisting of drops, ointments, lotions, gels, creams, patches, sprays, suspensions, and emulsions, but the present invention is not limited thereto.

Examples of carriers, excipients, and diluents that may be included in the dermatologic agent include lactose, dextrose, sucrose, oligosaccharides, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil.

In the dermatologic agent for the skin according to each formulation, components other than the composition of the present invention may be appropriately selected and mixed by those skilled in the art without difficulty depending on the formulations or use purposes of other dermatologic agents, and in this case, a synergistic effect may be obtained when applied simultaneously with other raw materials.

According to another embodiment of the present invention, there is provided a health functional food for improving skin whitening or thermal aging, which comprises 8-methoxybutin or a neutraceutically acceptable salt thereof as an active ingredient.

As used herein, "8-methoxybutin" is the same as described above.

The term "health functional food" as used herein refers to foods prepared and processed in the form of tablets, capsules, powder, granules, liquids, pills, or the like by using raw materials or ingredients having useful functionality in the human body. The term "functionality" as used herein refers to controlling nutrients for the structure and functions of the human body or providing useful effects of hygienic purposes, such as physiological actions, and the like. The health functional food of the present invention may be prepared by a method commonly used in the art and may be prepared by adding raw materials and ingredients that are generally added in the art. In addition, the health functional food may be prepared into any formulation without limitation as long as it is acceptable as a health functional food. The health functional food of the present invention may be prepared into various dosage forms, and unlike general pharmaceuticals, it is advantageous in that the health functional food uses foods as raw materials, thus not causing side effects that may occur during long-term administration of a drug, and has excellent portability, and thus the health functional food of the present invention may be ingested as a supplement for inhibiting thermal aging and improving a skin whitening effect.

In one embodiment of the present invention, as described above, 8-methoxybutin showed an effect of reducing tyrosinase activity, from which it was confirmed that the composition including 8-methoxybutin could be used as a health functional food for skin whitening (see FIG. 2).

In another embodiment of the present invention, as described above, it was confirmed that 8-methoxybutin inhibited melanin synthesis in a concentration-dependent manner (see FIG. 2), from which it was reconfirmed that the composition including 8-methoxybutin could be used as a health functional food for skin whitening.

In addition, in one embodiment of the present invention, as described above, 8-methoxybutin exhibited an effect of increasing the expression of heat shock protein 70, from which it was confirmed that the composition including 8-methoxybutin could be used as a health functional food for improving skin thermal aging (see FIG. 3).

The health functional food of the present invention may include various flavor enhancers, natural carbohydrates, or the like as additional ingredients. Non-limiting examples of the natural carbohydrates include monosaccharides such as glucose and fructose; disaccharides such as maltose and sucrose; polysaccharides such as dextrin and cyclodextrin; and sugar alcohols such as xylitol, sorbitol, and erythritol. As sweeteners, natural sweeteners such as thaumatin and stevia extracts or synthetic sweeteners such as saccharin and aspartame may be used.

In addition to the above-listed ingredients, the health functional food of the present invention may include various nutritional supplements, vitamins, electrolytes, flavors, colorants, pectic acid and salts thereof, alginic acid and salts thereof, a protective colloid thickener, a pH adjuster, a stabilizer, a preservative, glycerin, alcohols, a carbonating agent used in carbonated beverages, and the like. These ingredients may be used alone or a combination thereof may be used. The proportion of these additives is not very important, but the amounts of the additives generally range from 0.01 part by weight to 0.1 parts by weight with respect to 100 parts by weight of the health functional food of the present invention.

According to another embodiment of the present invention, there is provided a pharmaceutical composition for improving skin whitening or thermal aging, which comprises 8-methoxybutin or a pharmaceutically acceptable salt thereof as an active ingredient.

According to another embodiment of the present invention, there is provided a quasi-drug composition for improving skin whitening or thermal aging, which comprises 8-methoxybutin or a pharmaceutically acceptable salt thereof as an active ingredient.

As used herein, "8-methoxybutin" is the same as described above.

As used herein, the term "pharmaceutically acceptable salt" refers to a salt form in which 8-methoxybutin binds to other materials, and means a substance capable of exhibiting pharmaceutical activity similar to that of 8-methoxybutin.

In one embodiment of the present invention, as described above, 8-methoxybutin showed an inhibitory effect of tyrosinase activity, from which it was confirmed that the composition including 8-methoxybutin could be used as a pharmaceutical composition for skin whitening (see FIG. 2).

In another embodiment of the present invention, as described above, it was confirmed that 8-methoxybutin inhibited melanin synthesis in a concentration-dependent manner (see FIG. 2), from which it was reconfirmed that the composition including 8-methoxybutin could be used as a pharmaceutical composition for skin whitening.

In addition, in one embodiment of the present invention, as described above, 8-methoxybutin exhibited an effect of increasing the expression of heat shock protein 70, from which it was confirmed that the composition including 8-methoxybutin could be used as a pharmaceutical composition for improving skin thermal aging (see FIG. 3).

The pharmaceutical composition of the present invention may be formulated into forms such as tablets, pills, powders, granules, capsules, suspensions, liquids for internal use, emulsions, syrups, aerosols, or sterilized injections according to a general method for whitening, i.e., preventing or treating skin pigmentation.

Solid preparations for oral administration include tablets, pills, powders, granules, capsules, and the like, and such solid preparations are formulated by mixing the composition with at least one excipient, e.g., starch, calcium carbonate, sucrose, lactose, gelatin, and the like. In addition to simple excipients, lubricants such as magnesium stearate and talc are also used. Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a flavoring agent, a preservative, and the like.

Preparations for parenteral administration include an aqueous sterile solution, a non-aqueous solvent, a suspension, an emulsion, a freeze-dried preparation, a suppository, and the like. Non-limiting examples of the non-aqueous solvent and the suspension include propylene glycol, polyethylene glycol, a vegetable oil such as olive oil, and an injectable ester such as ethyl oleate.

In addition, the pharmaceutical composition of the present invention may further include a carrier, an excipient, or a diluent. As the carrier, the excipient, or the diluent, lactose, dextrose, sucrose, sorbitol, mannitol, xylitol, erythritol, maltitol, starch, acacia rubber, alginate, gelatin, calcium phosphate, calcium silicate, cellulose, methyl cellulose, hydroxyl propyl methyl cellulose, microcrystalline cellulose, polyvinylpyrrolidone, water, methyl hydroxybenzoate, propyl hydroxybenzoate, talc, magnesium stearate, mineral oil such as silicon dioxide, and the like may be used.

A suitable dose of the pharmaceutical composition according to the present invention may be variously selected by one of ordinary skill in the art according to factors such as formulation methods, the condition and body weight of a patient, the gender and age of a patient, the severity of disease, dosage form, administration route and period, excretion speed, reaction sensitivity, and the like, and the dose and the number of doses are not intended to limit the scope of the present invention in any way.

The pharmaceutical composition of the present invention may be administered to mammals such as rats, mice, livestock, and humans via various routes. All administration methods may be expected, and examples thereof may include oral injection, intravenous injection, intramuscular injection, and subcutaneous injection.

According to another embodiment of the present invention, there is provided a method of improving skin whitening or thermal aging, comprising applying, to the skin of an individual, a composition comprising 8-methoxybutin or a pharmaceutically acceptable salt thereof as an active ingredient. According to another embodiment of the present invention, there is provided a use of 8-methoxybutin or a pharmaceutically acceptable salt thereof for improving skin whitening or thermal aging.

The description of the 8-methoxybutin, and improvement of skin whitening and thermal aging has been provided above.

The composition of the present invention comprising 8-methoxybutin or a pharmaceutically acceptable salt thereof may be administered in any form or via any route, but when considering that the present invention is for improving skin conditions, the composition may be administered in the form of an external application for the skin. In addition, 8-methoxybutin or a pharmaceutically acceptable salt thereof may be administered or applied as it is, or may be administered or applied in the form of a composition.

The term "individual" as used herein refers to all animals including humans in need of improvement of skin conditions, particularly improvement of skin whitening or thermal aging, and particularly, may refer to all animals including mammals such as rats, mice, livestock, and humans.

### MODE OF INVENTION

Hereinafter, the present invention will be described in further detail with reference to the following examples. However, these examples are provided for illustrative purposes only and are not intended to limit the scope of the present invention.

### Example 1: Experimental Preparation

### 1-1: Cell Culture

HEK 293A and B16F10 cells used, Dulbecco's Modified Eagle's Medium (DMEM) containing 10% fetal bovine serum and 1% penicillin/streptomycin as a nutrient source, and were incubated in an incubator maintained at 37 °C under 5% CO₂.

### 1-2: Antibodies

Antibodies were purchased from respective manufacturers. Tyrosinase, 14-3-3 ζ, LAMIN B (Santa Cruz), MITF (Abcam), phospho-MITF (Sigma), ERK, phospho-ERK (Cell Signaling), ACTIN (MPBIO), and GAPDH (Bethyl) were used.

### Example 2: Confirmation of Effect of 8-Methoxybutin on Cytotoxicity and Cell Division

Since plant-derived natural substances can have potent toxicity in some cases and can affect cell division potential, the effects of 8-methoxybutin on toxicity and cell division were examined.

### 2-1: Cytotoxicity Experiment (MTT Experiment)

An MTT experiment was conducted to verify the stability of an 8-methoxybutin. This is an experiment for measuring the number of living cells through conversion of 3-(4,5-dimethyl-thiazol-2-yl)-2,5-diphenyl tetrazolium bromide (MTT) into formazan with a purple color, by reduction in cells. Cells were seeded into a 96-well cell culture plate at a density of 3 x 10³. After 24 hours, the medium was replaced with a culture medium containing 8-methoxybutin, followed by further incubation for 24 hours, and the cells were treated with MTT at a concentration of 5 mg/ml and incubated at 37 °C for 1 hour in a light-blocked state. Thereafter, the culture medium was removed, and a solvent for dissolving crystallized formazan was added to dissolve the sample, and the absorbance at 570 nm of the dissolved sample was measured.

### 2-2: Experimental Results

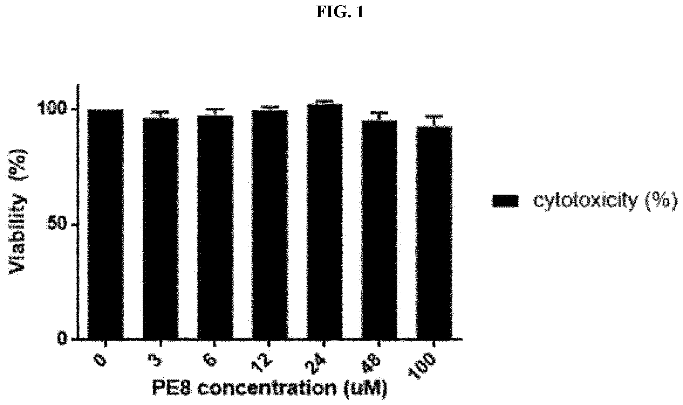

It was confirmed that the cell survival rate was maintained as high as about 100% even when treated with 8-methoxybutin at a high concentration as well as a low concentration. From these results, it was confirmed that, even upon treatment with 8-methoxybutin at a high concentration, i.e., 100 µM, cell division could occur actively and cytotoxicity was hardly observed (see FIG. 1).

### Example 3: Confirmation of Inhibition of Melanin Synthesis

To investigate the whitening effect of 8-methoxybutin, B16F10 cells were treated with 8-methoxybutin and the degree of activity of tyrosinase, which is a rate-limiting enzyme, and the degree of melanin synthesis were measured.

### 3-1: Experiment for Degree of Tyrosinase Activity at Cellular Level

B16F10 mouse melanoma cells were seeded into a 6-well cell culture plate at a density of 1 x 10⁵/well. The dispensed cells were treated with 8-methoxybutin, and then treated with 100 nM melanocyte stimulating hormone for 48 hours to induce melanin synthesis. The obtained cells were disrupted and then centrifuged to separate a supernatant. The protein concentration of the supernatant was measured, and 50 µg of protein per sample was standardized into a volume of 50 µl and placed on a 96-well plate. Then, 100 µl of 0.1% L-DOPA was added as a reaction substrate to each sample and allowed to react at 37 °C for 1 hour, and then absorbance at 475 nm was measured.

### 3-2: Experiment for Measuring Amount of Melanin

B16F10 mouse melanoma cells were seeded into a 6-well cell culture plate at a density of 1 x 10⁵/well. The dispensed cells were treated with 8-methoxybutin, and then treated with 100 nM melanocyte stimulating hormone (MSH) for 48 hours to induce melanin synthesis. The obtained cells were disrupted and then centrifuged to obtain a cell precipitate, and 1 N NaOH containing 10% DMSO was added thereto. Thereafter, the resulting precipitate was allowed to react at 60 °C for 1 hour, and absorbance at 405 nm was measured.

### 3-3: Experimental Results

When the B16F10 cells, treated with melanocyte stimulating hormone to induce melanin synthesis, were treated with 8-methoxybutin, the cells exhibited a decrease of 110% or more in tyrosinase activity and a decrease of 50% or more in melanin synthesis amount (see FIG. 2A). In addition, at this time, a remarkable decrease in the amount of tyrosinase protein was observed (see FIG. 2B), and it was confirmed at an mRNA level (see FIG. 2C). Next, while increasing the amount of 8-methoxybutin, tyrosinase activity and melanin synthesis were observed, and as a result, reductions in tyrosinase activity and melanin synthesis were shown in proportion to the amount of 8-methoxybutin (see FIG. 2D), and at this time, the amount of tyrosinase protein was also reduced in proportion to the amount of 8-methoxybutin (see FIG. 2E). From these results, it was confirmed that, upon treatment with 8-methoxybutin, tyrosinase activity was reduced, resulting in a reduced amount of melanin synthesized, thereby exhibiting a whitening effect.

### Example 4: Confirmation of Increase in Expression of Heat Shock Protein 70 (HSP70)

To determine the effect of 8-methoxybutin on improving thermal aging, heat stress was applied to skin cells and the amounts of heat shock protein 70 and heat shock protein 70 mRNA generated therefrom were measured.

### 4-1: Experimental Method

B16F10 cells treated with 8-methoxybutin and DMSO as a control, respectively, were subjected to thermal stress at 42 ° C for 1 hour. Total protein was extracted from the cells using a TNE buffer, and then the amount thereof was measured using a Bradford reagent. In addition, proteins were separated by SDS-PAGE and identified using antibodies, followed by quantitative analysis. Antibodies were purchased from respective manufacturers, and HSP70 (Santa Cruz) and actin (MPBIO) were used.

### 4-2: Experimental Results

Upon treatment with 8-methoxybutin, the amount of heat shock protein 70 (HSP70) was increased at least three times compared to a control under the heat-applied condition (at 42 ° C for 1 hour) (see FIG. 3A). In addition, a larger amount of heat shock protein 70 mRNA was observed in the 8-methoxybutin-treated sample than in the control (see FIG. 3B). From these results, it was confirmed that, upon treatment with 8-methoxybutin, the amount of heat shock protein 70 was increased, and thus thermal aging was inhibited, which is a skin improvement effect.

### Example 5: Confirmation of Phytoestrogenic Activity

### 5-1: RNA Extraction, cDNA Synthesis, RT-PCR and Quantitative Real-Time PCR

Total RNA was extracted from each group of cells using a TRI reagent (Molecular Research Center). Extracted RNA was reverse transcribed using an ImProm-TM Reverse Transcription System (Promega), followed by measurement using a StepOnePlusTM Real-Time PCR System (Applied Biosystems).

### 5-2: DNA Gene Transfer

To deliver a DNA plasmid into HEK 293A cells, a microporator (Invitrogen/Neon® Transfection System) was used. At one time, 1 µg of a reporter plasmid was delivered into 1 million cells. The DNA-delivered cells were cultured in a general culture medium.

### 5-3: Luciferase Experiment

The activities of Renilla and Firefly luciferases were measured using a Dual-Luciferase® Reporter Assay System (Promega). To measure the activity of an estrogen receptor (ER) reporter, a system, in which, when a specific material binds to the ER ligand domain, GAL4 binds to a specific DNA sequence, resulting in expression of the Firefly luciferase, was used, and the Renilla luciferase was delivered together to calibrate DNA transfer efficiency. The activity of the estrogen receptor reporter was determined by dividing a value of the Firefly luciferase by a value of the Renilla luciferase.

### 5-4: Experimental Results

First, the binding of 8-methoxybutin, which is a natural product-derived active substance, to the estrogen receptor was confirmed by a computer-binding prediction program (see FIG. 4).

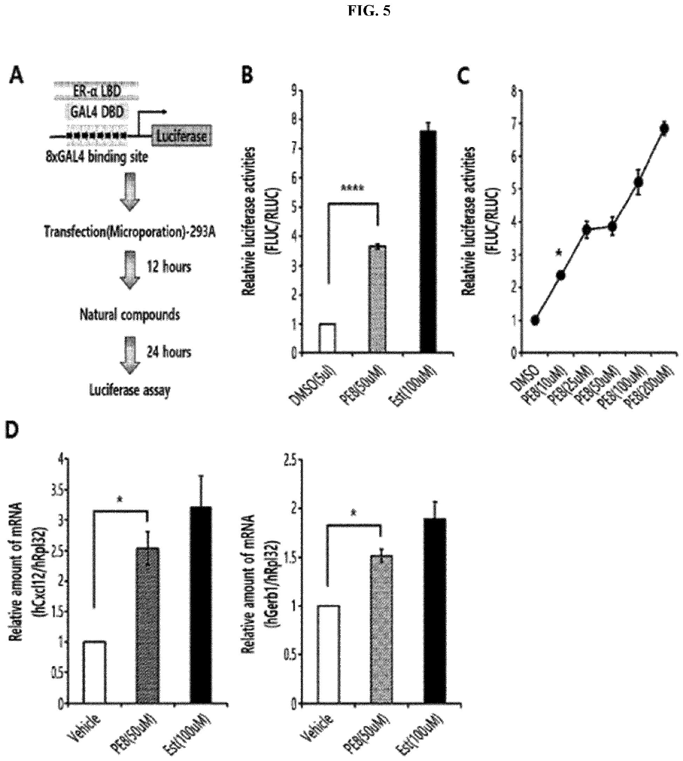

As a result of confirming the possibility that 8-methoxybutin binds to the estrogen receptor using a GAL4 luciferase reporter, the ERα ligand-binding domain is linked to the GAL4 DNA-binding domain, and when 8-methoxybutin binds to the ERα ligand-binding domain, the GAL4 DNA-binding domain binds to specific DNA, resulting in increased expression of the Firefly luciferase (see FIG. 5A). When HEK 293A cells to which reporter DNAs were delivered were treated with 50 µM of the extract, the expression of the luciferases was nearly 3.5 times higher than that of the control (see FIG. 5B). From these results, it was confirmed that 8-methoxybutin actually binds to the estrogen receptor in cells.

Next, as a result of confirming whether the expression of luciferases varies depending on the concentration of 8-methoxybutin, when the concentration was increased from 10 µM, a statistically significant increase in the expression of luciferases was shown (see FIG. 5C). In addition, when HEK 293A cells, which are known to express the estrogen receptor, were treated with 8-methoxybutin, the amount of mRNA of target genes was increased (see FIG. 5D), from which it was confirmed that actually, 8-methoxybutin acts as a ligand of the estrogen receptor in cells, and thus activates the estrogen receptor signaling pathway, and therefore, has an effect of improving skin aging.

From the foregoing description, it will be understood by one of ordinary skill in the art to which the present invention pertains that the invention may be embodied in various modified forms without departing from the technical spirit or essential characteristics thereof. Thus, the embodiments set forth herein should be considered in an illustrative sense only and not for the purpose of limitation in all aspects. The scope of the present invention should be defined only by the meanings and scope of the appended claims rather than the detailed description, and all changes or modifications derived from equivalents thereof should be construed as falling within the scope of the present invention.

## Claims

1. A cosmetic composition for improving skin whitening or thermal aging, the cosmetic composition comprising 8-methoxybutin or a cosmetically acceptable salt thereof as an active ingredient.

2. The cosmetic composition of claim 1, wherein the skin whitening is achieved by inhibiting tyrosinase activity.

3. The cosmetic composition of claim 1, wherein the improvement of skin thermal aging is achieved by increasing the expression of heat shock protein 70 (HSP70).

4. The cosmetic composition of claim 1, wherein the 8-methoxybutin has phytoestrogenic activity.

5. A dermatologic agent for improving skin whitening or thermal aging, the dermatologic agent comprising 8-methoxybutin or an acceptable salt thereof as an active ingredient.

6. A health functional food for improving skin whitening or thermal aging, the health functional food comprising 8-methoxybutin or a neutraceutically acceptable salt thereof as an active ingredient.

7. A pharmaceutical composition for improving skin whitening or thermal aging, the pharmaceutical composition comprising 8-methoxybutin or a pharmaceutically acceptable salt thereof as an active ingredient.

8. A quasi-drug composition for improving skin whitening or thermal aging, the quasi-drug composition comprising 8-methoxybutin or a pharmaceutically acceptable salt thereof as an active ingredient.

9. A method of improving skin whitening or thermal aging, the method comprising applying, to a skin of an individual, a composition comprising 8-methoxybutin or a pharmaceutically acceptable salt thereof as an active ingredient.
